# EUROPEAN PATENT SPECIFICATION

(11) **EP 0 404 936 B1**
(45) Date of publication and mention of the grant of the patent: **25.05.1994**
(21) Application number: 89903225.4
(22) Date of filing: 14.03.1989
(51) Int. Cl.: C07C 401/00

(54) **PREPARATION OF VITAMIN D2 AND VITAMIN D3, OR ACTIVE VITAMIN D2 AND ACTIVE VITAMIN D3, OR THEIR DERIVATIVES**
HERSTELLUNG VON VITAMIN D2 UND VITAMIN D3 ODER AKTIVEM VITAMIN D2 UND AKTIVEM VITAMIN D3 ODER IHREN DERIVATEN
PREPARATION DE VITAMINE D2 et D3, OU DE VITAMINE ACTIVE D2 ET D3 OU DE LEURS DERIVES

(30) Priority: 16.03.1988 JP 62517/88
(43) Date of publication of application: 02.01.1991
(73) Proprietor: DAIDO HOXAN INC., Chuo-ku Sapporo (JP)
(72) Inventor: TAHARA, Yuji Hoxan Laboratory, Shiroishi-ku, Sapporo-shi Hokkaido 003 (JP)
(74) Representative: Gibson, Stewart Harry
(86) International application number: JP8900274
(87) International publication number: WO8908641

(56) References cited:
- JP-A-59 212 466
- JOURNAL OF ORGANIC CHEMISTRY, vol. 51, no. 8, 18th April 1986, pages 1269-1272,American Chemical Society; J.L. MASCARENAS et al.: "Studies on the synthesis ofside-chain hydroxylated metabolites of vitamin D. 3. Synthesis of 25-ketovitamin D3 and 25-hydroxyvitamin D3"

## Description

### FIELD OF THE INVENTION

This invention relates to a method of manufacturing vitamin D₂, vitamin D₃, activated type vitamin D₂, activated type vitamin D₃ and their derivatives from 7,8-dihydroxy vitamin D₂, 7,8-dihydroxy vitamin D₃ and their derivatives.

### BACKGROUND OF THE INVENTION

There has been a remarkable development of study of vitamin D since 1958 when 25-hydroxy vitamin D₃ was found as an activated metabolite of vitamin D₃ and more extensively since 1971 when 1α,25-dihydroxy vitamin D₃ which is by far the most activated substance of all the known metabolites of vitamin D₃ was discovered. (See the following reference papers.)
- Reference Paper 1:: H.F. DeLuca et al., Ann. Rev. Biochem., vol.45, p.631, 1976
- Reference Paper 2:: H.F. DeLuca, Nutr. Rev., vol.37, p.161, 1979
Since then extensive researches have been done in the fields of biochemistry, organic chemistry, and medicine through cooperation of researchers of these and other research areas. As a result of the research efforts, 1α,25-dihydroxy vitamin D₃ which is an activated type vitamin D₃ and its homologue 1α,25-hydroxy vitamin D₃ were developed as medicines for kidney diseases, bone diseases and thyroidal disorders. (See the following reference papers.)
- Reference Paper 3:: D.E.M. Lawson, "vitamin D", Academic Press, Inc., New York, 1978
- Reference Paper 4:: A.W. Norman, "Vitamin D, The Calcium Homeostasis Steroid Hormone", Academic Press, Inc., New York, 1979
It has been known for some time that 1α,25-dihydroxy vitamin D₃ is particularly effective for small intestine and bone and more recently there was a report evidencing that kidney, pancreas, pituitary body, thymus, thyroid, skin, mammary gland, lymphocyte and many other tissues and organs have receptors for the chemical. (See Reference Paper 5 below.) Likewise, it has also been proved that various tumorous cells such as malignant melanoma cells (see Reference Paper 6 below), breast cancer cells (see Reference Paper 7 below), osteogenic sarcoma cells (see Reference Paper 8 below) and myeloid leukemia cells (see Reference Paper 9 below) have receptors.
- Reference Paper 5:: H.F. DeLuca et al., Proc. Natl.Acas. Sci.USA, vol.77, p.1149, 1980
- Reference Paper 6:: K. Colston et al., Endocrinology, vol.108, p.1083, 1981
- Reference Paper 7:: H.F. Freake et al., Biochem.Biophys. Res.Commun., vol.101, p.1131, 1981
- Reference Paper 8:: S.C. Mnolagas et al., J.Biol.Chem., vol.255, p.4414, 1980
- Reference Paper 9:: T. Suda et al., Biochem.J., vol.204, p.713, 1982
Particularly the fact that 1α,25-dihydroxy vitamin D₃ suppresses proliferation of myeloid leukemia cells and accelerates differentiation of cells (see Reference Paper 10 below) and that there is a certain relationship between activated type vitamin D and the immune system of the body suggests potential and encouraging applications of the chemical (see Reference Paper 11 below).
- Reference Paper 10:: T. Suda et al., Proc.Natl,Acad.Sci., USA, vol.78, p.4990
- Reference Paper 11:: E. Abe, Vitamin, vol.59, p.418, 1985
Since the 1α-hydroxy component of activated type vitamin D and its various derivatives is considered to play a vital and fundamental role in the physiological effects of these chemicals, many laboratories and research institutes all over the world have been concentrating their efforts on the study of the component and consequently a number of achievements have been reported in the research field. Some of the examples of the achievements of the researches include,
1) a photochemical method of obtaining desired vitamin D derivatives in which 1α-hydroxidated steroid is synthesized in the first place and then it is converted to the corresponding 1α-hydroxy-5,7-dienesterol derivative which is used as material for the vitamin derivative,
   - Reference Paper 12:: C. Kaneko, Organic Synthetic Chemistry, vol.33, p.75, 1975, etc.
2) a method with which a vitamin D derivative is converted to a 3,5-cyclovitamin derivate and then combined with allylic acid at position C(1), the product being converted again to obtain the desired vitamin D derivative,
   - Reference Paper 13:: H.F. DeLuca et al., J. Org. Chem., vol.45, p.3253, 1980 etc.
3) a method with which vitamin D is temporarily converted to transvitamin D, which is combined with allylic acid at position C(1), the product being photochemically reconverted to vitamin D and
   - Reference Paper 14:: R.H. Hesse et al., J. Org. Chem., vol.51, p.1635, p.4819, 1986 etc.
4) a method with which a fragment corresponding to an A-ring portion and having a hydroxyl group at position C(1) is synthesized with a view to total synthesis and then combined with a fragment that corresponds to a D-ring portion to obtain the aimed compound.
   - Reference Paper 15:: W.H. Okamura et al., Tetrahedron Letters, vol.28, p.2095, 1987
   - Reference Paper 16:: E.G. Baggiolini et al., J.Org.Chem., vol.51, p.3098, 1986
   - Reference Paper 17:: L. Castedo et al., Tetrahedron Letters, vol.28, p.2099, 1987 etc.

Of the above cited methods, the method 1) is most popularly used as it is a relatively practical one, although it comprises a number of steps required to introduce a hydroxyl group to position 1α and its stereo- and positional selectivity is not very good, making the overall process rather inefficient as a number of steps are further required to obtain the aimed final vitamin D derivative by means of conversion. On the other hand, the method 2) allows use of any known process for conversion from vitamin D to 3,5-cyclovitamin D with a high yield (see the paper below).
- Reference Paper 18:: Y. Mazur et al., J. Am. Chem. Soc., vol.97, p.6249, 1979
The reaction with allylic acid of the method 2) above has a high stereoselectivity although it can not be conducted with a high yield as oxo compounds are brought forth at position C(1) as by-product at a significant level. Moreover, the method (b) is accompanied by other problems such as the fact that by-products including transvitamin D, which are not easily separable from the aimed 1α-hydroxy vitamin D, are produced in the solvolysis of the 1α-hydroxy compound.

The method (3) also has problems because it entails a low yield in the oxidation stage or because a photochemical process is required for conversion of the oxidized compound into vitamin D, although it has a high conversion yield from vitamin D to transvitamin D.

Finally, the method (4) is unsatisfactory in practice because of the number of steps required.

As is apparent from the above description, known methods are not satisfactory and development of more effective and efficient methods is desired.

We have now developed an effective synthetic method for producing significant intermediate compounds that can be combined with allylic acid at position C(1) for production of vitamin D and also developed an effective and efficient method which is based on a concept and procedures completely different from those of existing methods and in which vitamin D₂, vitamin D₃, activated type vitamin D₂, activated type vitamin D₃ or any of their derivatives is obtained from 7,8-dihydroxy vitamin D₂, D₃ or any of their derivatives by means of a reducing elimination technique involving a cyclic ortho-ester or a thiocarbonate of a 7,8-dihydroxy compound as an intermediate compound or an elimination technique using a reducing metal such as titanium.

The present invention comprises a method of manufacturing vitamin D₂, vitamin D₃, activated type vitamin D₂, activated type vitamin D₃ or derivatives thereof, expressed by formula [ II ] below 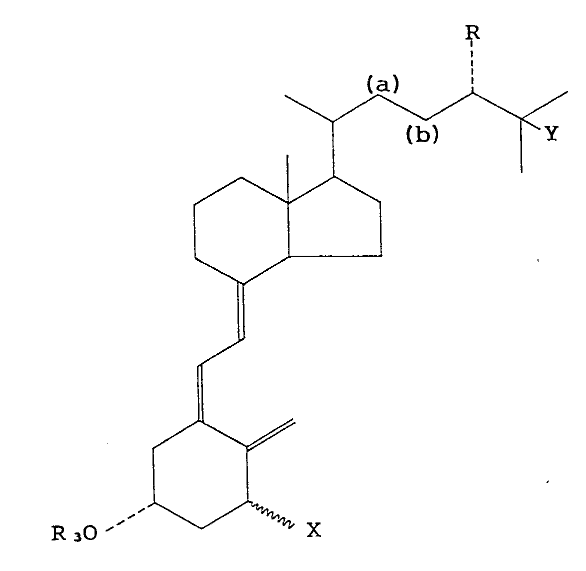
where R represents a hydrogen atom or methyl group;
the carbon-carbon bond between atoms (a) and (b) of the side chain is either a single or double carbon-carbon bond;
R₃ represents a hydrogen atom or a hydroxy protecting group; and each of X and Y represents a hydrogen atom, a hydroxy group or a derivative thereof;
wherein 7,8-dihydroxy vitamin D₂ or D₃ or derivatives thereof, expressed by formula [ I ] below 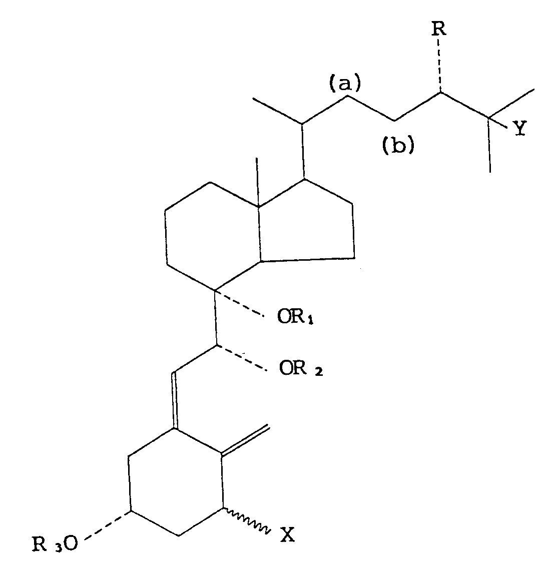
wherein R, R₃, X, Y and the carbon-carbon bond between atoms (a) and (b) of the side chain are as defined with reference to Formula (II), and
R₁ and R₂, which may be the same or different, each represents a hydrogen atom or a hydroxy protecting group;
is subjected to reductive elimination by way of a cyclic orthoester, or a thiocarbonate or an elimination reaction by means of a reducing metal, such as titanium.

The method according to the invention can be used for effectively manufacturing the above cited chemical compounds of formula I on an industrial basis.

Preferred compounds expressed by formula I above are as follows:
1. 7,8-dihydroxy-7,8-dihydro vitamin D₃ (R, R₁, R₂, R₃, X and Y = H; a single bond between atoms (a) and (b)),
2. 3β-O-(t-butyldimethylsilyl)-7,8-dihydroxy-7,8-dihydro vitamin D₃ (R, R₁, R₂, X and Y = H; R₃ = Si-t-BuMe₂; a single bond between atoms (a) and (b) - see Reference Paper 19, i.e. W.H. Okamura et al., J. Org. Chem., Vol.48, p.1414, 1983).
3. 7,8-dihydroxy vitamin D₂ (R₁, R₂, R₃, X and Y = H; R = Me; a double bond between atoms (a) and (b) see Reference Paper 20, i.e. Y. Wang et al., Acta. Chem Sin., Vol.24, p. 126, 1958).
4. Any of the derivatives of 7,8,25-trihydroxy vitamin D₃ (R, X = H; R₁, R₂ and R₃ = H or a hydroxy protecting group; Y = OR₄, wherein R₄ = H or a hydroxy protecting group; a single bond between atoms (a) and (b) - see Reference Paper 21, i.e. H.F. DeLuca et al., Japanese Patent Application No. 59-93130).

Isolation of the desired chemical from the mixture of reaction products can be conducted very easily. In one known isolation process, the reaction product containing vitamin D₂, vitamin D₃ or any of their active type homologues is dissolved in toluene with excess of orthoethylformate and an amount of camphor sulphonic acid as catalyst and the mixture is stirred at room temperature to form a cyclic orthoester. Then, prior to isolation, the mixture is heated in a Dean-Stark apparatus at reflux and thereafter the solvent is removed from the mixture by distillation. The residue is refined by means of column chromatography to obtain pure vitamin D₂, vitamin D₃ or any of their derivatives at a high yield.

Now the present invention will be described in greater detail by way of examples. In the following description, vitamin D refers to vitamin D₂ and vitamin D₃ and the spectrum values represent those of vitamin D₃.

### [Example 1]

### "Preparation of t-butyldimethylsilyl vitamin D"

100mg of 3β-O-(t-butyldimethylsilyl)-7,8-dihydroxy-7,8-dihydro vitamin D and 100mg of triethylorthoformate were dissolved in 20mℓ of toluene and an amount of camphor sulfonic acid as catalyst was added to the solution while it was being stirred at room temperature. The solution was further stirred for 30 minutes at the same temperature and then heated in a Dean-Stark apparatus for four hours at reflux. After completion of reaction, the solvent was removed by distillation and the residue was subjected to a silica gel column chromatography process (silica gel 1g, solvent; n-hexane) to obtain 67mg of vitamin D combined with t-butyldimethylsilyl.

The chemical structure of the product was identified by comparing its IR and NMR spectrums with those of a specimen producted by directly combining vitamin D and t-butyldimethylsilyl.

The reaction formulas are shown below.

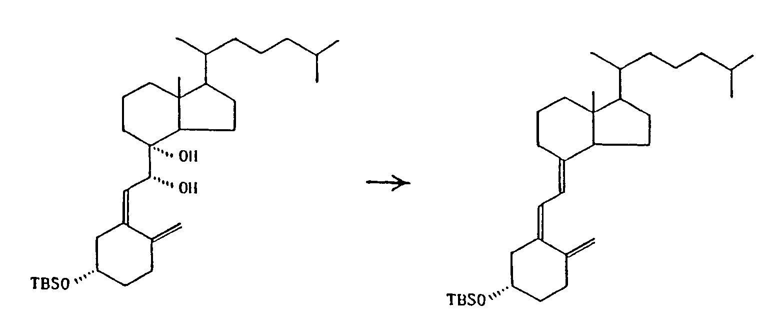

### [Example 2]

### "Preparation of 1β-acetoxy-(t-butyldimethylsilyl) vitamin D"

100mg of 1β-acetoxy-3β-O-(t-butyldimethylsilyl)-7,8-dihydroxy-7,8-dihydro vitamin D and 100mg of triethylorthoformate are dissolved in 20mℓ of toluene with an amount of camphor sulfonic acid as catalyst and the solution was heated for two hours in a Dean-Stark apparatus at reflux. After completion of reaction, the solvent was removed by distillation and the residue was subjected to a silica gel column chromatography process (silica gel 1g, solvent; n-hexane) to obtain 50mg of 1β-acetoxy-(t-butyldimethylsilyl) vitamin D.
IR spectrum: ν max (CHCℓ₃) cm⁻¹: 1720
NMR spectrum: (CCℓ₄) δ : 0.10(6H,S), 0.57(3H,S), 0.87(9H, d,J=6Hz), 0.93(9H,S), 2.10(3H,S), 3.55-4.10(1H,m), 4.87(1H, brs), 4.80-5.15(1H,m), 5.15(1H,m), 5.15(1H,brs), 5.85(1H,d, J=12Hz), 6.23(1H,d,J=12Hz)
mass spectrum (FD)m/e: 590(M⁻), 589, 573, 498, 438, 378
The reaction formulas are shown below.

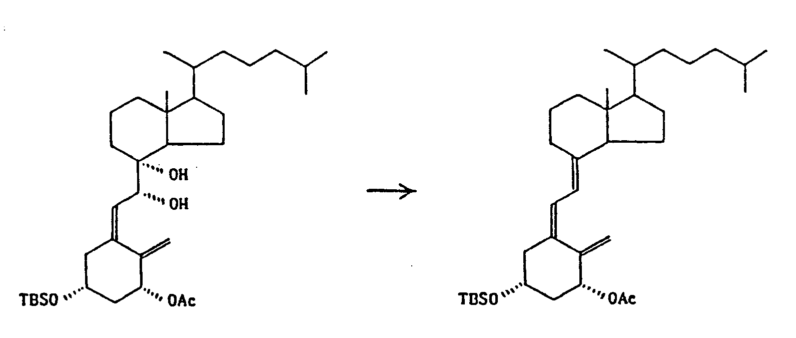

### [Example 3]

### "Preparation of 1β-benzoyloxy-3β-O-(t-butyldimethylsilyl)-7,8-dihydroxy-7,8-dihydro vitamin D"

200mg of 3β-O-(t-butyldimethylsilyl)-7,8-dihydroxy-7,8-dihydro-1β-hydroxy vitamin D and 21mℓ of pyridine were dissolved in 5mℓ of dry methylene chloride with an amount of 4-dimethylaminopyridine as catalyst and 70mg of benzoyl chloride was added to the solution while it was being stirred at 0°C . After completion of reaction, the solution was diluted with 30mℓ of methylene chloride while it was being stirred at room temperature and then washed sequentially with water, 10% hydrochloric acid, saturated aqueous solution of sodium hydrogencarbonate and water. After drying the solution with sodium sulfate and removing the solvent by distillation, the residue was subjected to a silica gel chromatography process [silica gel 4g, solvent; n-hexane-ethyl acetate (100:5 v/v)] to obtain 200mg of 1β-benzoyloxy-3β-O-(t-butyldimethylsilyl)-7,8-dihydroxy-7,8-dihydro vitamin D.
IR spectrum: ν max (CHCℓ₃) cm⁻¹: 3550, 1710
NMR spectrum: (CCℓ₃) δ : 0.10(6H,S), 0.75(3H,S), 0.85(9H, d,J=6Hz), 0.90(9H,S), 3.40-3.90(1H,m), 4.75(1H,d,J=10Hz), 5.10(2H,brs), 5.10-5.30(1H,m), 5.70(1H,d,J=10Hz), 7.30-7.60 (3H,m), 7.93-8.20(2H,m)
mass spectrum (FD)m/e: 652(M⁻), 635, 617, 595, 577, 560, 530, 513, 473, 442
The reaction formulas are shown below.

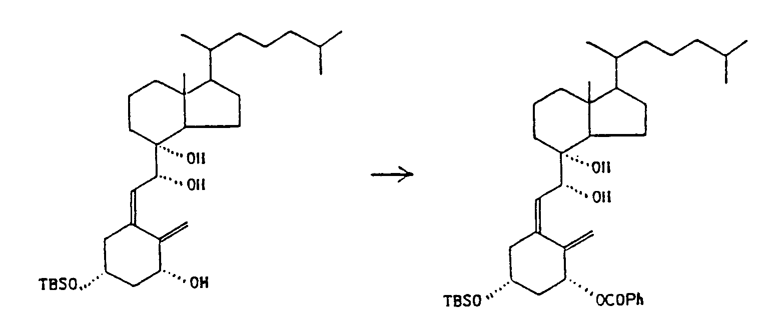

### [Example 4]

### "Preparation of 1β-benzoyloxy-(t-butyldimethylsilyl) vitamin D"

100mg of 1β-benzoyloxy-O-(t-butyldimethylsilyl)-7,8-dihydroxy-7,8-dihydro vitamin D and 100mg of triethylorthoformate were dissolved in 20mℓ of dry toluene with an amount of pyridinium-para-toluenesulfonate catalyst and the solution was heated in a Dean-Stark apparatus for ten minutes at reflux. After completion of reaction, the solvent was removed by distillation and the residue was subjected to a silica gel column chromatography process [silica gel 1g, solvent; n-hexane-benzene (100:1 v/v)] to obtain 50mg of 1β-benzoyloxy-(t-butyldimethylsilyl) vitamin D.
IR spectrum: ν max (CHCℓ₃) cm⁻¹: 1715
NMR spectrum: (CCℓ₄) δ : 0.10(6H,S), 0.55(3H,S), 0.88(9H, d,J=6Hz), 0.90(9H,S), 3.60-4.20(1H,m), 4.93(1H,brs), 5.30 (1H,brs), 5.20-5.60(1H,m), 5.86(1H,d,J=12Hz), 6.26(1H,d, J=12Hz), 7.30-7.60(3H,m), 7.97-8.20(2H,m)
mass spectrum (FD)m/e: 6.18(M⁻), 496, 119
The reaction formulas are shown below.

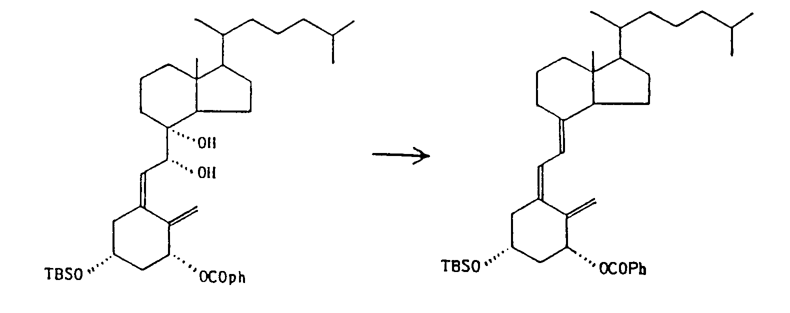

### [Example 5]

### "Preparation of 3β-O-(t-butyldimethylsilyl)-7,8-dihydroxy-7.8-dihydro-1β-methoxycarbonyloxy vitamin D"

100mg of 3β-O-(t-butyldimethylsilyl)-7,8-dihydroxy-7,8-dihydro-1β-hydroxy vitamin D and 1mℓ of pyridine are dissolved in 2mℓ of dry methylene chloride with an amount of 4-dimethylaminopyridine as catalyst and then 100mg of methyl chlorocarbonate was added to the solution while it was being stirred at 0°C. The solution was further stirred for two hours at room temperature and diluted with 20mℓ of methylene chloride. The diluted solution was washed sequentially with water, 10% hydrochloric acid, saturated aqueous solution of sodium hydrogencarbonate and water and thereafter dried with sodium sulfate.

After removing the solvent by distillation, the residue was subjected to a silica gel column chromatography process [silica gel 1g, solvent; n-hexane-ethyl acetate (100:5 v/v)] to obtain 100mg of 3β-O-(t-butyldimethylsilyl)-7,8-dihydroxy-7,8-dihydro-1β-methoxycarbonyloxy vitamin D.
IR spectrum: ν max (CHCℓ₃) cm⁻¹: 3550, 1740
NMR spectrum: δ : 0.09(6H,S), 0.77(3H,S), 0.87(9H,d,J=6Hz), 0.90(9H,S), 3.80(3H,S), 3.50-3.90(1H,m), 3.80(3H,S), 4.60-4.90(1H,m), 4.70(1H,d,J=10Hz), 5.10(2H,brs), 5.65(1H,d, J=10Hz),
mass spectrum (FD)m/e: 606(M⁻), 605, 589, 572, 549, 531, 513, 473, 455, 448, 380
The reaction formulas are shown below.

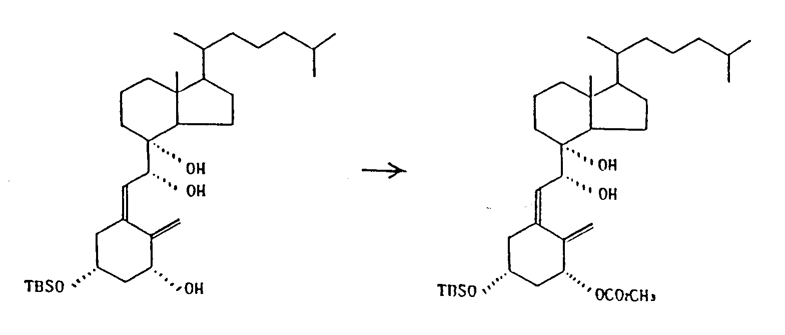

### [Example 6]

### "Preparation of 1β-methoxycarbonyloxy-(t-butyldimethylsilyl) vitamin D"

90mg of 3β-O-(t-butyldimethylsilyl)-7,8-dihydroxy-7,8-dihydro-1β-methoxycarbonyloxy vitamin D and 90mg of triethylorthoformate were dissolved in 20mℓ of dry toluene with an amount of pyridinium-para-toluenesulfonate as catalyst and the solution was heated in a Dean-Stark apparatus for 30 minutes at reflux. After completion of reaction, the solvent was removed by distillation and the residue was subjected to silica gel column chromatography [silica gel 1g, solvent; n-hexane-benzene (100:1 v/v)] to obtain 50mg of 1β-methoxycarbonyloxy-(t-butyldimethylsilyl) vitamin D.
IR spectrum: ν max (CHCℓ₃) cm⁻¹:1740
NMR spectrum: (CCℓ₄) δ : 0.09(6H,S), 0.56(3H,S), 0.85(9H, d,J=6Hz), 0.90(9H,S), 3.70-4.00(1H,m), 3.80(3H,S), 4.80-5.10(1H,m), 4.90(1H,brs), 5.25(1H,brs), 5.86(1H,d,J=10Hz), 6.25(1H,d,J=10Hz)
mass spectrum (FD)m/e: 572(M⁻), 408, 276
The reaction formulas are shown below.

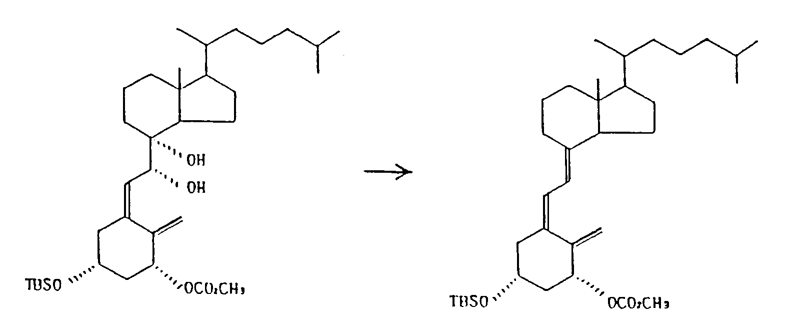

### [Example 7]

### "Preparation of 1-β-acetoxy-7,8-dihydroxy-7,8-hydro vitamin D"

120mg of 1β-acetoxy-3β-O-(t-butyldimethylsilyl)-7,8-dihydroxy-7,8-dihydro vitamin D was dissolved in 1mℓ of methanol with an amount of p-toluenesulfonate as catalyst and the solution was stirred for two hours at room temperature. After completion of reaction, the solvent was removed by distillation and the residue was dissolved in chloroform and washed with saturated aqueous solution of sodium hydrogencarbonate. Again the solvent was removed by distillation and the residue was washed with n-hexane to obtain 100mg of β-acetoxy-7,8-dihydroxy-7,8-dihydro vitamin D.
IR spectrum: ν max (CHCℓ₃) cm⁻¹: 3550, 1740
NMR spectrum: (CDCℓ₃) δ : 0.77(3H,S), 0.85(9H,d,J=6Hz), 2.17(3H,S), 3.70-4.10(1H,m), 4.80(1H,d,J=10Hz), 5.13(1H, brs), 4.90-5.30(1H,m), 5.80(1H,d.J=10Hz)
mass spectrum (FD)m/e: 476(M⁻), 459, 416
The reaction formulas are shown below.

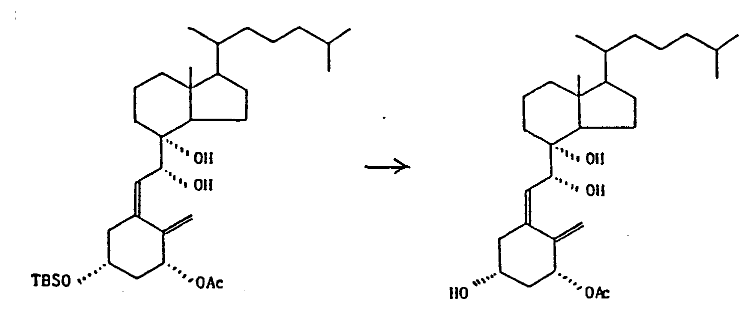

### [Example 8]

### "Preparation of 1β-acetoxy-3β-O-acetyl-7,8-dihydroxy-7,8-dihydro vitamin D"

100mg of 1β-acetoxy-7,8-dihydroxy-7,8-dihydro vitamin D was dissolved in a mixture of 2mℓ of pyridine and 5mℓ of methylene chloride and 26mg of acetic anhydride was dropped into the solution, which was then stirred for 30 minutes at room temperature. After completion of reaction, the solution was diluted with 10mℓ of methylene chloride and washed sequentially with 10% hydrochloric acid, water, saturated aqueous solution of sodium hydrogencarbonate and water. The solution was dried with sodium sulfate and the solvent was removed by distillation. The residue was then subjected to a silica gel column chromatography process [silica gel 1g, solvent; n-hexane-ethyl acetate (10:2 v/v)] to obtain 100mg of 1β-acetoxy-3β-O-acetyl-7,8-dihydroxy-7,8-dihydro vitamin D.
IR spectrum: ν max (CHCℓ₃) cm⁻¹: 3550, 1740
NMR spectrum: (CCℓ₄) δ : 0.74(3H,S), 0.85(9H,d,J=6Hz), 1.97 (3H,S), 2.07(3H,S) 4.40-4.90(1H,m), 4.63(1H,d,J=10Hz), 4.95-5.10(1H,m), 4.98(1H,brs), 5.03(1H,brs), 5.65(1H,d, J=10Hz)
mass spectrum (FD)m/e: 518(M⁻), 501, 458, 265
The reaction formulas are shown below.

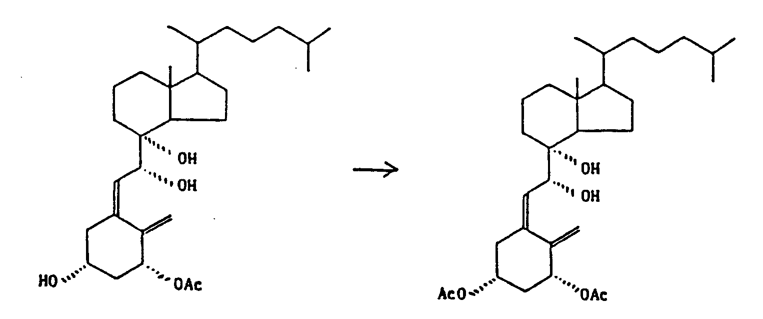

### [Example 9]

### "Preparation of 1β-acetoxy-3β-O-acetyl vitamin D"

90mg of 1β-acetoxy-3β-O-acetyl-7,8-dihydroxy-7,8-dihydro vitamin D was dissolved in 20mℓ of dry toluene and an amount of pyridinium-para-toluenesulfonate as catalyst was added to the solution while it was being stirred. The solution was then heated in a Dean-Stark apparatus for 30 minutes at reflux. After completion of reaction, the solvent was removed by distillation and the residue was subjected to a silica gel column chromatography process [silica gel 1g, solvent; n-hexane-benzene (2:3 v/v)] to obtain 70mg of 1β-acetoxy-3β-O-acetyl vitamin D.
IR spectrum: ν max (CHCℓ₃) cm⁻¹: 1730
NMR spectrum: (CCℓ₄) δ : 0.55(3H,S), 0.90(9H,d,J=6Hz), 1.95(3H,S), 2.05(3H,S), 4.80-5.00(1H,m), 4.90(1H,m), 4.90 (1H,brs), 5.20(1H,brs), 5.20-5.50(1H,m), 5.90(1H,d,J=10Hz), 6.30(1H,d,J=10Hz)
mass spectrum (FD)m/e: 484(M⁻), 422, 398
The reaction formulas are shown below.

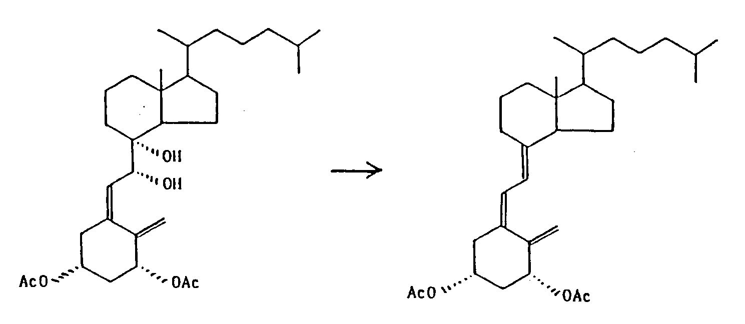

### [Example 10]

### "Preparation of 1β-acetoxy vitamin D"

100mg of 1β-acetoxy-3β-O-t-butyldimethylsilyl vitamin D was dissolved into a mixture of 2mℓ of methanol and 2mℓ of methylenechloride with in the presence of P-toluenesulfonate as catalyst and the solution was stirred for two hours at room temperature. After completion of reaction, the solution was diluted with 10mℓ of methylene chloride and washed sequentially with water, saturated aqueous solution of sodium hydrogencarbonate and water. Then the diluted solution was washed with sodium sulfonate and the solvent was removed by distillation. Thereafter, the residue was subjected to a silica gel column chromatography process [silica gel 1g, solvent; n-hexane-ethyl acetate (100:5 v/v)] to obtain 90mg of 1β-acetoxy vitamin D.
IR spectrum: ν max (CHCℓ₃) cm⁻¹: 1730
UV λ max (EtOH)nm: 264, 244
NMR spectrum: (CDCℓ₃) δ : 0.53(3H,S), 0.87(9H,d,J=6Hz), 2.07(3H,S), 3.70-4.20(1H,m), 5.0(1H,brs), 5.27(1H,brs), 5.20-5.50(1H,m), 5.90(1H,d,J=11Hz), 6.37(1H,d,J=11Hz)
mass spectrum (FD)m/e: 442(M⁻), 383
The reaction formulas are shown below.

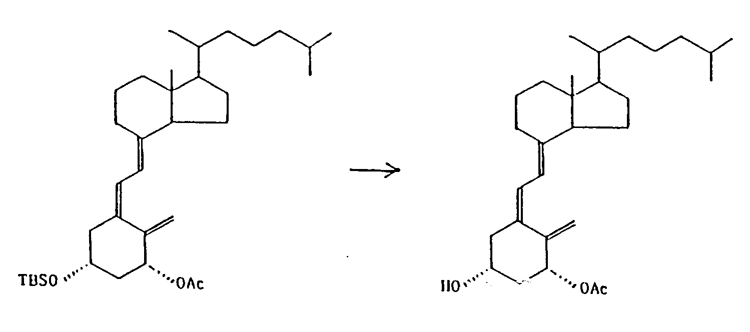

### [Example 11]

### "Preparation of 1β-benzoyloxy vitamin D"

A 80mg of 1β-benzoyloxy-3β-O-(t-butyldimethylsilyl) vitamin D was dissolved in a mixture of 2mℓ of methanol and 2mℓ of methylene chloride with in the presence of p-toluenesulfonate as catalyst and the solution was stirred for two hours at room temperature. After completion of reaction, the solution was diluted with 10mℓ of methylene chloride and washed sequentially with water, saturated aqueous solution of sodium hydrogencarbonate and water. Thereafter, the solvent was removed by distillation and the residue was subjected to a silica gel column chromatography process [silica gel 1g, solvent; n-hexane-ethyl acetate (10:1 v/v)] to obtain 50mg of 1β-benzoyloxy vitamin D.
IR spectrum: ν max (CHCℓ₃) cm⁻¹: 1720
NMR spectrum: (CCℓ₄) δ : 0.53(3H,S), 0.86(9H,d,J=6Hz), 3.70-4.60(1H,m), 5.00(1H,brs), 5.38(1H,brs), 5.40-5.70 (1H,m), 5.87(1H,d,J=12Hz), 6.36(1H,d,J=12Hz), 7.30-7.60 (3H,m), 7.90-8.20(2H,m)
mass spectrum (FD)m/e: 504(M⁻), 382, 264
The reaction formulas are shown below.

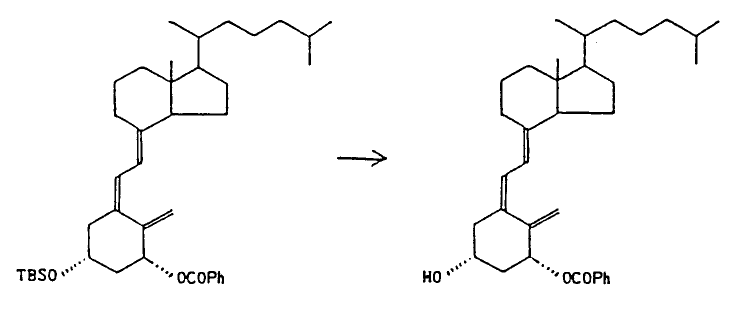

### [Example 12]

### "Preparation of 1β-hydroxy vitamin D"

28mg of 1β-acetoxy vitamin D was dissolved in 2mℓ of methanol, to which 0.1mℓ of caustic soda containing 10% methanol was added. The solution was then stirred for one hour at room temperature and, after completion of reaction, diluted with 10mℓ of methylene chloride. Thereafter, the solution was washed with water and dried with anhydrous potassium carbonate. After the solvent was removed, the residue was subjected to a silica gel column chromatography process [silica gel 1g, solvent; n-hexane-ethyl acetate (1:1 v/v)] to obtain 20mg of 1β-hydroxy vitamin D.

30mg of 1β-hydroxy vitamin was obtained from 40mg of 1β-benzoyloxy vitamin D following a similar process.
IR spectrum: ν max (CHCℓ₃) cm⁻¹: 3500
UV ν max (EtOH)nm: 264, 244
NMR spectrum: (CDCℓ₃) δ : 0.57(3H,S), 0.90(9H,d,J=6Hz), 3.85-4.20(1H,m), 4.20-4.50(1H,m), 5.00(1H,brs), 5.30 (1H,brs) 6.05(1H,d,J=10Hz), 6.47(1H,d,J=10Hz)
mass spectrum (FD)m/e: 400(M⁻), 382, 364
The reaction formulas are shown below. 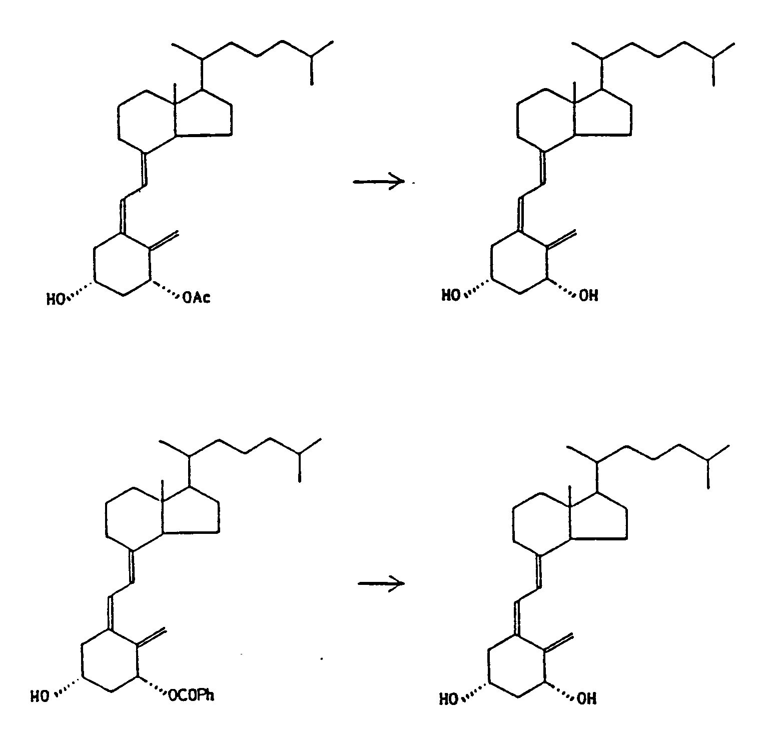
While a variety of 1β-hydroxy vitamin D derivatives were synthesized by using various 7,8-dihydroxy-7,8-dihydro vitamin D derivatives in the above twelve examples, 1α-hydroxy vitamin D derivatives were synthetically prepared from 1β-hydroxy vitamin D derivatives in the following examples.

### [Example 13]

### "Preparation of 3β-(t-butyldimethylsilyl)-1β-hydroxy vitamin D"

300mg of 1β-benzoyloxy-3β-O-(t-butyldimethylsilyl) vitamin D were dissolved in a mixture of 2mℓ of methanol and 2mℓ of methylene chloride, to which caustic soda solution containing methanol by 10% was added. The solution was stirred for 30 minutes at room temperature and, after completion of reaction, diluted with 20mℓ of methylene chloride. Then, the diluted solution was washed with water and dried with anhydrous potassium carbonate. Thereafter, the solvent was removed by distillation and the residue was subjected to a silica gel column chromatography process [silica gel 5g, solvent; n-hexane-ethyl acetate (100:1 v/v)] to obtain 227mg of 3β-O-(t-butyldimethylsilyl)-1β-hydroxy vitamin D.
IR spectrum: ν max (CHCℓ₃) cm⁻¹: 3450
NMR spectrum: (CCℓ₄) δ : 0.10(6H,S), 0.55(3H,S), 0.86(9H, d,J=6Hz), 0.90(9H,S), 3.86-4.20(2H,m), 4,80(1H,brs), 5.15 (1H,brs), 5.85(1H,d,J=12Hz), 6.15(1H,d,J=12Hz)
mass spectrum (FD)m/e: 514(M⁻), 496
The reaction formulas are shown below.

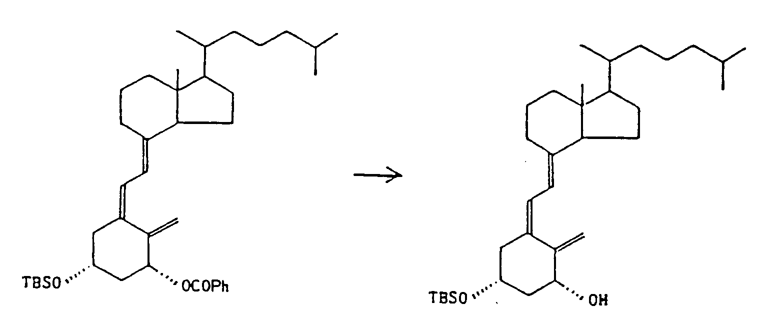

### [Example 14]

### "Preparation of 1α-benzoyloxy-3β-O-t-butyldimethylsilyl vitamin D"

93mg of 3β-O-(t-butyldimethylsilyl)-1β-hydroxy vitamin D, 56.9mg of triphenylphosphine and 26.5mg of benzoic acid were dissolved in 2mℓ of anhydrous benzene, to which 37.8mg of diethyl-azo-dicarboxylate was added. The solution was stirred for three hours at room temperature and, after completion of reaction, diluted with 10mℓ of benzene. Thereafter, it was washed sequentially with 10% hydrochloric acid, water, saturated aqueous solution of sodium hydrogencarbonate and water and dried with sodium sulfate. The solvent was removed by distillation and the residue was subjected to a silica gel column chromatography process [silica gel 1g, solvent; n-hexane-acetic acid (100:1 v/v)] to obtain 1α-benzoyloxy-3β-O-(t-butyldimethylsilyl) vitamin D.
IR spectrum: ν max (CHCℓ₃) cm⁻¹: 1710
NMR spectrum (CCℓ₄) δ : 0.10(6H,S), 0.30(3H,S), 0.86(9h,d, J=6Hz), 0.90(9H,S), 3.90-4.20(1H,m), 5.00(1H,brs), 5.35 (1H,brs), 5.40-5.70(1H,m), 5.40(1H,d,J=12Hz), 6.23(1H,d, J=12Hz), 7.10-7.45(3H,m)), 7.70-8.00(2H,m)
mass spectrum (FD)m/e: 618(M⁻), 593, 578, 562, 547, 528, 512, 483, 464, 436
The reaction formulas are shown below.

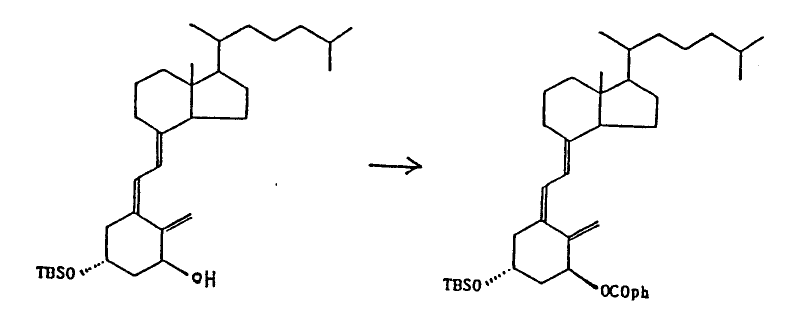

### [Example 15]

### "Preparation of 3β-O-(t-butyldimethylsilyl)-1α-hydroxy vitamin D"

30mg of 1α-benzoyloxy-3β-O-(t-butyldimethylsilyl) vitamin D was dissolved in a mixture of 1mℓ of methanol and 10mℓ of methylene chloride, to which 0.1mℓ of caustic soda containg methanol by 10% was added. The solution was heated and stirred for one hour. After completion of reaction, the solution was diluted with 20mℓ of methylene chloride and washed with water. Thereafter, it was dried with anhydrous potassium carbonate and the solvent was removed by distillation. Then the residue was subjected to a silica gel column chromatography process [silica gel 1g, solvent; n-hexane-ethylacetate (100:5 v/v)] to obtain 25mg of 3β-O-(t-butyldimethylsilyl)-1α-hydroxy vitamin D.
IR spectrum: ν max (CHCℓ₄) cm⁻¹: 3550
NMR spectrum: (CCℓ₄) δ : 0.07(6H,S), 0,.55(3H,S), 0.87 (9H,d,J=6Hz), 0.90(9H,S), 3.90-4.50(2H,m), 4.86(1H,brs), 5.17(1H,brs), 5.84(1H,d,J=12Hz), 6.17(1H,d,J=12Hz)
mass spectrum (FD)m/e: 514(M⁻)
The reaction formulas are shown below.

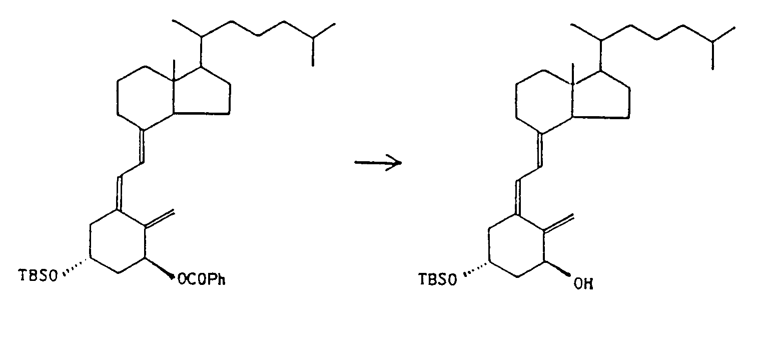

### [Example 16]

### "Preparation of 1α-hydroxy vitamin D"

A 30mg of 3β-O-(t-butyldimethylsilyl)-1α-hydroxy vitamin D was dissolved into 10mℓ of anhydrous tetrahydrofuran, to which 0.1mℓ of tetra-n-butylammoniumfluoride containing 1mol of tetrahydrofuran was added. The solution was heated and stirred for one hour. After completion of reaction, the solvent was removed by distillation and the residue was washed with water. Thereafter, it was dried with anhydrous potassium carbonate and the solvent was further removed by distillation. The residue was subjected to a silica gel column chromatography process [silica gel 1g, solvent; benzene-ethyl acetate (3:2 v/v)] to obtain 23g of 1α-hydroxy vitamin D.
m.p. 139-140°C, Colorless Needles (from petroleum ether)
IR spectrum ν max (CHCℓ₃) cm⁻¹: 3600
UV ν max (EtOH)nm: 264, 244
NMR spectrum: (CDCℓ₃) δ : 0.57(3H,S), 0.92(9H,S), 0.92(9H, d,J=6Hz), 4.00-4.60(2H,m), 5.02(1H,brs), 5.34(1H,brs), 6.02(1H,d,J=12Hz), 6.42(1H,d,J=12Hz)
mass spectrum (FD)m/e: 400(M⁻)
The reaction formulas are shown below. 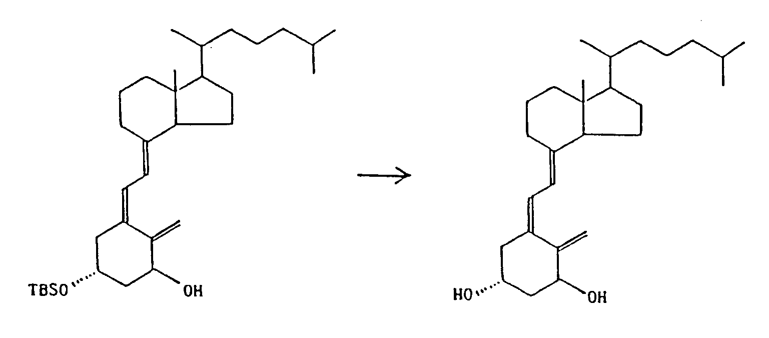
The above described Examples 13 through 16 are examples of variations of 1α-hydroxy vitamin D obtained from various 1β-hydroxy vitamin D derivatives.

### POTENTIAL INDUSTRIAL APPLICATIONS

As described above, the present invention provides a method with which vitamin D₂, vitamin D₃, activated type vitamin D₂, activated type vitamin D₃ and their derivatives are prepared from 7,8-dihydroxy vitamin D₂, D₃ or their derivatives by means of a reducing elimination technique involving a cyclic orthoester of a 7,8-dihydroxy compound or a thiocarbonate compound as an intermediate compound or an elimination technique utilizing a reducing metal such as titanium. The method according to the invention ensures a practical and simple synthetic process and a high yield as compared with any existing methods for manufacturing these chemicals.

## Claims

1. A method of manufacturing vitamin D₂, vitamin D₃, activated type vitamin D₂, activated type vitamin D₃ or derivatives thereof, expressed by formula [ II ] below, 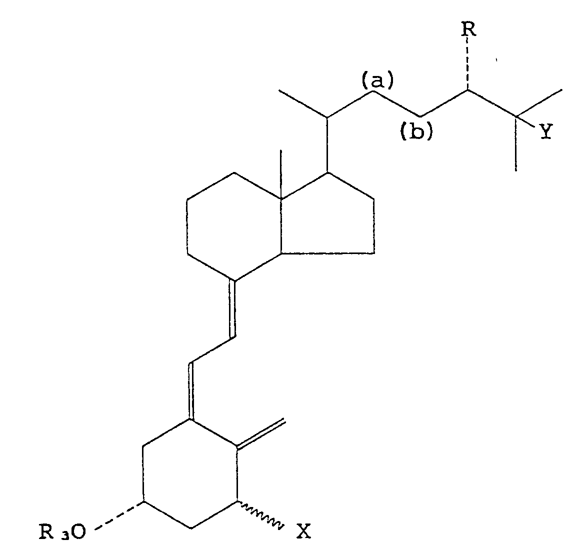 where R represents a hydrogen atom or methyl group;
the carbon-carbon bond between atoms (a) and (b) of the side chain is either a single or double carbon-carbon bond;
R₃ represents a hydrogen atom or a hydroxy protecting group; and each of X and Y represents a hydrogen atom, a hydroxy group or a derivative thereof;
wherein 7,8-dihydroxy vitamin D₂ or D₃, or derivatives thereof, expressed by formula (I) below 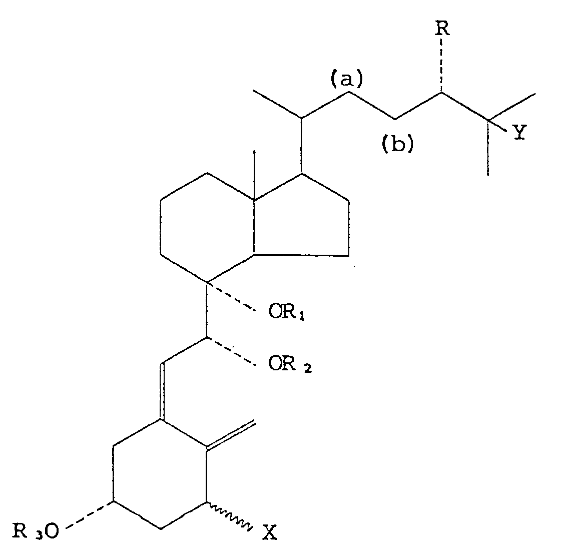 wherein R, R₃, X, Y and the carbon-carbon bond between atoms (a) and (b) of the side chain are as defined with reference to formula (II);
R₁ and R₂, which may be the same or different, each represents a hydrogen atom or a hydroxy protecting group;
is subjected to reductive elimination by way of a cyclic orthoester or a thiocarbonate, or an elimination reaction by means of a reducing metal.

2. A method according to claim 1, wherein said reducing metal comprises titanium.

## Patentansprüche

1. Verfahren zur Herstellung von Vitamin D₂, Vitamin D₃, aktiviertem Typ von Vitamin D₂, aktiviertem Typ von Vitamin D₃ oder Derivaten davon der nachstehenden Formel [II] 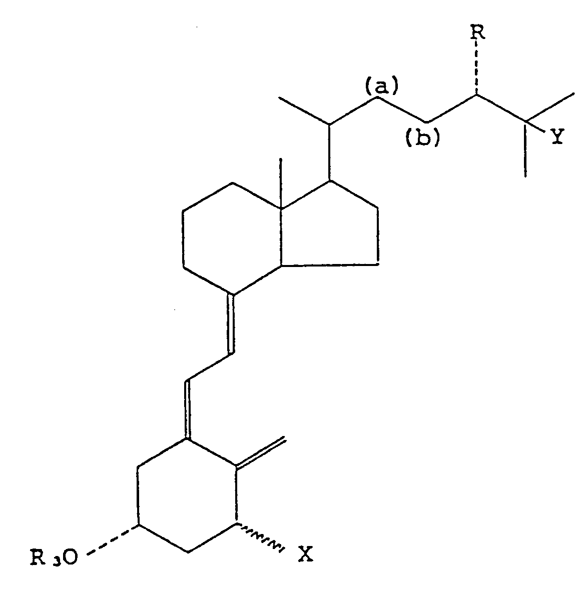 worin R ein Wasserstoffatom oder eine Methylgruppe bedeutet;
es sich bei der Kohlenstoff-Kohlenstoff-Bindung zwischen den Atomen (a) und (b) der Seitenkette entweder um eine Kohlenstoff-Kohlenstoff-Einfach-oder -Doppelbindung handelt;
R₃ ein Wasserstoffatom oder eine Hydroxylschutzgruppe bedeutet; und die einzelnen Reste X und Y jeweils ein Wasserstoffatom, eine Hydroxylgruppe oder ein Derivat davon bedeuten; wobei 7,8-Dihydroxy-vitamin D₂ oder D₃ oder Derivate davon der nachstehenden Formel (I) 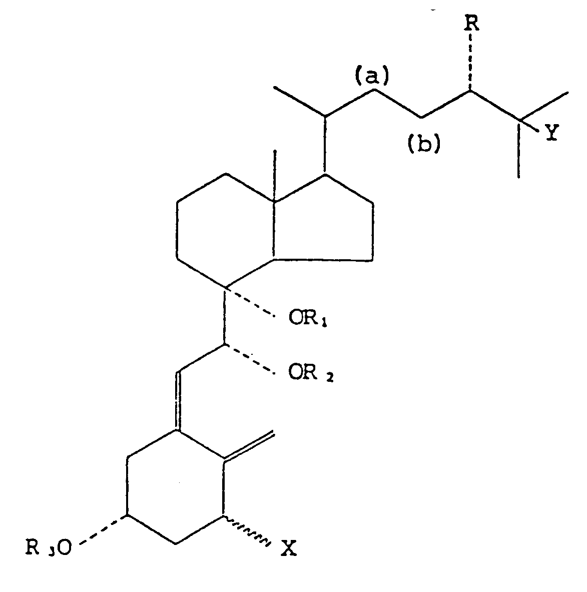 in der R, R₃, X, Y und die Kohlenstoff-Kohlenstoff-Bindung zwischen den Atomen (a) und (b) der Seitenkette die in bezug auf die Formel (II) angegebene Definition aufweisen; und R₁ und R₂, die gleich oder verschieden sein können, jeweils ein Wasserstoffatom oder eine Hydroxylschutzgruppe bedeuten, einer reduktiven Elimination mittels eines cyclischen Orthoesters oder eines Thiocarbonats oder einer Eliminationsreaktion mittels eines reduzierenden Metalls unterworfen werden.

2. Verfahren nach Anspruch 1, wobei das reduzierende Metall Titan umfaßt.

## Revendications

1. Procédé de préparation de vitamine D₂, de vitamine D₃, de vitamine active D₂, de vitamine active D₃ ou de leurs dérivés, répondant à la formule (II) ci-dessous : 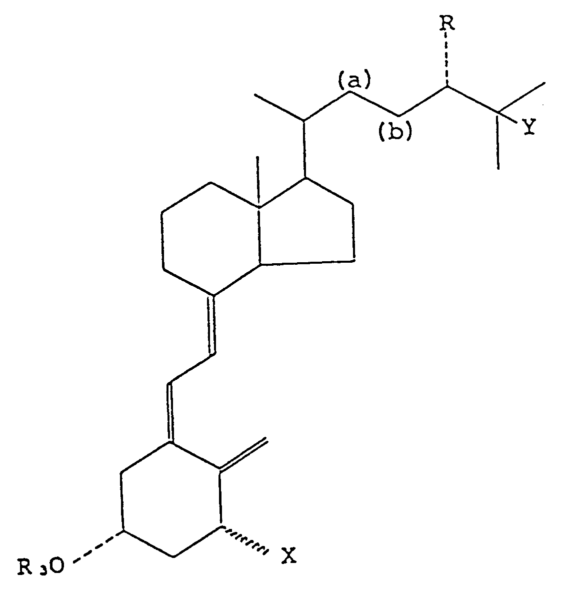 dans laquelle R représente un atome d'hydrogène ou un groupe méthyle;
la liaison carbone-carbone entre les atomes (a) et (b) de la chaine latérale est soit une simple liaison soit une double liaison carbone-carbone;
R₃ représente un atome d'hydrogène ou un groupe hydroxy-protecteur; et chaque X et Y représente un atome d'hydrogène, un groupe hydroxyle ou un dérivé de celui-ci;
dans lequel la 7,8-dihydroxy vitamine D₂ ou D₃, ou un dérivé de celles-ci, répondant à la formule (I) ci-dessous 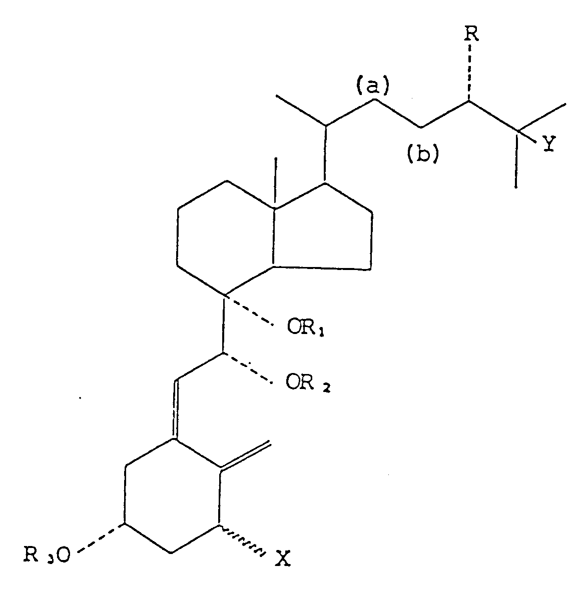 dans laquelle R, R₃, X, Y et la liaison carbone-carbone entre les atomes (a) et (b) de la chaine latérale sont tels que définis en référence à la formule (II);
R₁, et R₂, qui peuvent être identiques ou différents, représentent chacun un atome d'hydrogène ou un groupe hydroxy-protecteur;
est soumis à une élimination réductrice grâce à un thiocarbonate ou à un orthoester cyclique, ou à une réaction d'élimination grâce à un métal réducteur.

2. Procédé selon la revendication 1, dans lequel ledit métal réducteur comprend du titane.
